# EUROPÄISCHE PATENTSCHRIFT

(11) **EP 1 514 873 B1**
(45) Veröffentlichungstag und Bekanntmachung des Hinweises auf die Patenterteilung: **19.03.2008**
(21) Anmeldenummer: 04013059.3
(22) Anmeldetag: 03.06.2004
(51) Int. Cl.: C07F 13/00, B01J 23/36

(54) **Verfahren zur Oxidation organischer Substrate und Katalysatorzusammensetzung zur Durchführung des Verfahrens**
Process for oxidation of organic substrates and catalyst compositions for performing the same
Procédé pour oxydation de composés organiques et composés catalytiques pour son performation

(30) Priorität: 12.09.2003 DE 10342150
(43) Veröffentlichungstag der Anmeldung: 16.03.2005
(73) Patentinhaber: Evonik Degussa GmbH, 40474 Düsseldorf (DE)
(72) Erfinder: Riermeier, Thomas, Dr., 65439 Flörsheim (DE); Hoff, Manfred, 65779 Kelkheim (DE); Drauz, Karlheinz, Prof., 63579 Freigericht (DE); Zeiss, Werner, Dr., 82547 Eurasburg (DE); Wahren, Uwe, Dr., 86153 Augsburg (DE)

(56) Entgegenhaltungen:
- US-A- 5 155 247
- US-A- 5 939 568
- HERMANN ET AL: "Multiple Bonds between Main-Group Elements and Transition Metals" ORGANOMETALLICS, Bd. 11, 1992, Seiten 1072-1081, XP002294045
- STRAVROPOULOS, PERICLES ET AL: "Oxoaryls of rhenium(V) and -(VI) and osmium(VI). X-ray crystal structures of dimesityldioxorhenium(VI), tetramesityloxorhenium(VI), and dimesityldioxoosmium(VI)" JOURNAL OF THE CHEMICAL SOCIETY, DALTON TRANSACTIONS: INORGANIC CHEMISTRY (1972-1999) , (1), 169-75 CODEN: JCDTBI; ISSN: 0300-9246, 1987, XP002294046

## Beschreibung

Die Erfindung betrifft ein Verfahren zur Oxidation organischer Substrate, wobei diese in einem Lösungsmittelsystem in Gegenwart einer OrganylrheniumoxidVerbindung als Katalysator mit einer Peroxoverbindung, insbesondere Wasserstoffperoxid oder einer Quelle für Wasserstoffperoxid, umgesetzt werden. Erfindungsgemäß wird eine Katalysatorzusammensetzung eingesetzt, wie sie bei der Organylierung einer Rheniumverbindung der allgemeinen Formel ReOₘAₙ, worin A eine anionische Abgangsgruppe ist, mit einer Organometallverbindung ohne Isolierung und Reinigung der im Reaktionsgemisch enthaltenen Organylrheniumoxidverbindung erhältlich ist. Weiterer Gegenstand ist eine unter Verwendung einer Grignardverbindung erhaltene Katalysatorzusammensetzung zur Durchführung des Verfahrens.

Organylrhenium(VII)oxide, insbesondere Methylrheniumtrioxid, das meist kurz als MTO bezeichnet wird, haben in jüngerer Zeit zunehmend Interesse als Katalysatoren für Oxidationsreaktionen gefunden, wie die Oxidation von Olefinen zu Epoxiden und Diolen (US 5,166,372), Oxidation von Aromaten zu Chinonen (DE 44 197 99), Oxidation von aromatischen Aminen zu Nitrosoverbindungen bzw. N-Oxiden und Isomerisierungen und die Metathese von Olefinen (WO 91/14665). Eine Übersicht über die Herstellung, Strukturen und Anwendungen von Rhenium(VII)Oxo- und Imido-Komplexen vermitteln C.C.Romäo und W.A. Herrmann et al. in Chem. Rev. 1997,97, 3197-3246.

Zur Herstellung von Alkylrheniumtrioxid, wie MTO als Grundkörper der Organylrheniumtrioxide, haben sich drei Synthesewege etabliert: Ein erster Weg umfasst die Direktalkylierung von Dirheniumheptoxid (Re₂O₇) mit Tetraalkylzinn oder Dialkylzink (W.A. Herrmann, J. Organomet, Chem. 1995, 500, 149 - 174). Hierbei werden allerdings 50% des Rheniums in unreaktives Trialkylstannylperrhenat bzw. Zinkperrhenat überführt. Ein wesentlicher Nachteil dieses Verfahrens ist, dass die an sich sehr wirksamen Alkylzinnverbindungen hochtoxisch sind. Ein zweiter Weg umfasst die Alkylierung von Re₂O₇ in Gegenwart von Trifluoressigsäureanhydrid, wodurch zwar die Bildung rheniumhaltiger Nebenprodukte, also der Perrhenate, weitgehend vermieden wird, andererseits aber das wasserempfindliche und teure Anhydrid das Verfahren verteuert. Auch hier werden wieder im allgemeinen toxische Zinnalkyle zur Alkylierung eingesetzt.

Schließlich lässt sich gemäß Herrmann et al. (Angew. Chem. 1997,109,2767-2768) MTO in einem dritten Verfahren ohne Feuchtigkeitsausschluss durch Umsetzung von Perrhenaten der Formel M(ReO₄) oder M'(ReO₄)₂, worin M ein Alkalimetall, NH₄, Ag oder (CH₃)₃Sn und M' Zn oder Ca ist, mit Tetramethylzinn und Trimethylchlorsilan erzeugen. Nachteilig ist wiederum das Erfordernis, ein flüchtiges hochtoxisches Alkylierungsmittel einsetzen zu müssen. Ferner muss das MTO enthaltende Reaktionsgemisch filtriert, dann unter vermindertem Druck vom Lösungsmittel befreit und schließlich das Roh-MTO durch Sublimation im Hochvakuum gereinigt werden, wodurch sich die Herstellung verteuert.

Gemäß WO 98/47907 lassen sich nach dem vorgenannten Verfahrensprinzip generell katalytisch wirksame Verbindungen der Formel RₐRe_{b}O_{c}L_{d} erzeugen, worin R ein Organylrest, a eine ganze Zahl von 1 bis 6, b eine ganze Zahl von 1 bis 4, c eine ganze Zahl von 1 bis 12, d eine ganze Zahl von 0 bis 4 und L ein Lewis-basischer Ligand und Re fünf- bis siebenwertig ist. Als organylierende Reagenzien werden in diesem Dokument Organometallverbindungen von Sn, Zn, Al, Mg, Li, Cu, Cd und Hg, vorzugsweise aber solche von Sn und Zn, genannt. Auch wenn Organometallverbindungen des Magnesiums erwähnt werden, gibt es keinen Hinweis darauf, eine Grignardverbindung als alleiniges Organylierungsmittel einzusetzen. Dieses Dokument lehrt nämlich nur, dass die bevorzugt einzusetzenden Zinkalkylierungsmittel (ZnR₂) durch eine situ-Reaktion aus ZnCl₂ und Alkyllithium (RLi) oder einer Grignardverbindung (RMgX) gebildet werden können. Die Notwendigkeit, sowohl ein Zinksalz und zusätzlich eine Grignardverbindung einsetzen zu müssen, verteuert aber das Verfahren. Obgleich die bei der Aufarbeitung von Reaktionsgemischen zwecks Rückgewinnung von Re-Katalysatoren anfallenden Perrhenate vor der Organylierung nicht gereinigt werden müssen, wird das als Katalysator einzusetzende Organylrheniumoxid selbst durch Sublimation oder durch Kristallisation gereinigt.

Auch bei der äthenolytischen Metathese von Olefinen gemäß WO 91/14665 wird ein Rheniumkatalysator der vorgenannten Gattung RₐRe_{b}O_{c}, wie (CH₃)ReO₃ oder (CH₃)₄Re₂O₄, hier allerdings an einen oxidischen Träger gebunden, stets in gereinigter Form eingesetzt. Hinweise, eine bei der Herstellung des Organylierungsmittels erhaltene Zusammensetzung ohne aufwendige Reinigungsoperation als Katalysatorzusammensetzung zu verwenden, lassen sich diesem Dokument nicht entnehmen.

Bezüglich der Verwendung von Rheniumkatalysatoren der zuvor genannten allgemeinen Formel RₐRe_{b}O_{c}L_{d} als Oxidationskatalysator wird auch auf das EP-Patent 0380 085 verwiesen. Das in diesem Dokument angegebene Herstellverfahren für den einzusetzenden Katalysator MTO oder aminischen Komplex von MTO umfasst wiederum eine Vakuumsublimation des MTO. Alternativ zu MTO wird festes Tetramethyltetraoxodirhenium ((CH₃)₄Re₂O₄) als Ausgangsverbindung eingesetzt.

Im Beispiel 42 der EP 0 380 085 B1 kommt (Trimethylsilyl)methylmagnesiumchlorid, also eine Grignardverbindung, zur Anwendung: Diese Verbindung wird mit dem Bipyridinkomplex von Methyl(oxo)dichlororhenium(V) umgesetzt; das von Lösungsmitteln befreite Reaktionsgemisch wird chromatographisch aufgetrennt und der Trimethyloxorheniumkomplex aus der entsprechenden Fraktion kristallisiert. Hinweise, ein Reaktionsgemisch durch eine Umsetzung einer Oxorheniumverbindung der Formel ReOₘAₙ mit einem Grignardreagenz zu erzeugen und dieses ohne aufwendige Reinigungsoperation, ausgenommen Filtration oder Extraktion und Abdestillieren von Lösungsmittel, als Katalysator in Oxidationsreaktionen, bei der Metathese von Olefinen und in anderen mit Organylrheniumoxiden katalysierten Reaktionen zu verwenden, lassen sich diesem Dokument nicht entnehmen.

In dem einleitend zitierten Review-Artikel (Chem. Rev. 1997) wird auf Seite 3210 dargelegt, dass von der anionischen Substitution von Verbindungen vom Typ XReO₃, wie ClReO₃, Re₂O₇ oder Me₃SiOReO₃, mit Lithium-, Magnesium- und Aluminiumalkylen abgeraten wird, weil es hiermit in stärkerem Umfang als mit Zinkalkylen zu Reduktionsreaktionen kommt. Schließlich wird auf Seite 3211 dieses Dokuments gelehrt, dass das einem Grignard-Reagenz strukturgleiche Isopropylzinkchlorid gegenüber Dirheniumheptoxid unreaktiv ist. Eine Anregung zur alleinigen Verwendung von Methylmagnesiumchlorid oder Methylzinkchlorid oder alpha-unverzweigten Alkylmagnesiumhalogeniden oder entsprechenden Alkylzinkhalogeniden als Organylierungsmittel und Verwendung eines Alkylrheniumoxid enthaltenden Reaktionsgemischs als Katalysator in Oxidationsreaktionen wird nicht gegeben.

Gemäß W.A. Herrmann und F.E. Kühn (Acc. Chem. Res. 1997, 30, 169 - 180) lassen sich Organorhenium(VII)oxide auch durch katalytische Oxidation von Organorheniumalkylen mit H₂O₂ und Jod als Katalysator erzeugen. In diesem Dokument wird auch gelehrt, dass unter Verwendung von Grignard-Reagenzien oder Alkyllithium aus OReCl₃(PPh₃)₂ oder OReCl₄, jeweils mit nachfolgender Oxidation der Re(V)Zwischenstufe, Alkylrhenium(VI)oxide der Formel R₄ReO erhältlich sind. Einer Anregung, eine Verbindung vom Typ ReOₘAₙ, worin Re siebenwertig und A eine anionische Abgangsgruppe ist, mittels einer Grignardverbindung zu alkylieren, lässt sich diesem Dokument nicht entnehmen.

Aufgabe der vorliegenden Erfindung ist es demgemäß, ein weiteres Verfahren zur Durchführung von Oxidationsreaktionen organischer Substrate in Gegenwart von Organylrheniumoxiden und Derivaten davon aufzuzeigen, wobei ein in einfacher Weise erhältlicher Rheniumkatalysator zur Anwendung gelangen sollte, um den Gesamtaufwand für die Katalysatorbereitstellung und die Oxidationsreaktion zu reduzieren.

Aufgabe eines weiteren Gegenstands der Erfindung ist es, eine Katalysatorzusammensetzung, enthaltend ein Organylrheniumoxid, aufzuzeigen, die ohne aufwendige Reinigungsoperationen in Oxidationsreationen eingesetzt werden kann.

Es wurde gefunden, dass für die fraglichen Oxidationsreaktionen mit Persauerstoffverbindungen, wie insbesondere Wasserstoffperoxid oder einer Quelle hierfür, eine Katalysatorzusammensetzung, wie sie im Rahmen der Herstellung der Organylrheniumoxide erhältlich ist, ohne nennenswerte Reinigungsoperationen, insbesondere keine Sublimation, Destillation oder Kristallisation des Organylrheniumoxids, überraschender Weise als Katalysator im Wesentlichen ebenso wirksam ist, wie eine äquivalente Menge des bisher eingesetzten aufwendig gereinigten Katalysators. Bisher wurde offensichtlich angenommen, dass auf die aufwendigen Reinigungsoperationen der Organylrheniumoxide, wie MTO, nicht verzichtet werden kann, ohne eine entsprechende Verschlechterung der katalytischen Wirksamkeit des Katalysators in Kauf nehmen zu müssen.

Gegenstand der Erfindung ist ein Verfahren zur Oxidation organischer Substrate, umfassend Umsetzung des organischen Substrats in einem Lösungsmittelsystem mit einer Peroxoverbindung, insbesondere Wasserstoffperoxid, in Gegenwart eines Organylrheniumoxids der Formel RₐRe_{b}O_{c}L_{d}, worin R ein Organylrest, a eine ganze Zahl von 1 bis 6, b eine ganze Zahl von 1 bis 4, c eine ganze Zahl von 1 bis 12, d eine ganze Zahl von 0 bis 4, L ein Lewis-basischer Ligand und Re fünf- bis siebenwertig ist, als Katalysator, dadurch gekennzeichnet, dass man eine Katalysatorzusammensetzung K1 oder eine daraus gewonnene Katalysatorzusammensetzung K2, K3 oder K4 einsetzt, wobei die Herstellung von K1 die Umsetzung einer Oxorheniumverbindung der allgemeinen Formel ReOₘAₙ, worin Re fünf- bis siebenwertig ist, A für eine anionische Abgangsgruppe, m für die Zahl 1, 2 oder 3 und n für die Zahl 0, 1, 2 oder 3 stehen, mit 0,5 bis 10 Organyläquivalenten pro Mol ReOₘAₙ eines Organylierungsmittels eines Elements aus der Reihe Li, Na, K, Mg, Al und Zn in einem aprotischen Lösungsmittel in An- oder Abwesenheit eines Lewis-basischen Liganden umfasst, die Herstellung von K2 die Zugabe eines protischen Mittels zu einer überschüssiges Organylierungsmittel enthaltenden Zusammensetzung K1 und die Herstellung von K3 bzw. K4 mindestens teilweises Entfernen von Lösungsmitteln und/oder anorganischen Bestandteilen aus K1 bzw. K2 umfasst, die Herstellung von K1 bis K4 aber eine Sublimation, Kristallisation oder Destillation des oder der darin enthaltenen Organylrheniumoxide der Formel RₐRe_{b}O_{c}L_{d} ausschließt.

Die Unteransprüche richten sich auf bevorzugte Ausführungsformen des Verfahrens im Hinblick auf die Herstellung der zu verwendenden Katalysatorzusammensetzung und die Oxidation spezieller Substrate.

Ein wesentliches Kennzeichen des erfindungsgemäßen Verfahrens besteht darin, dass zur Durchführung der Oxidation eine Katalysatorzusmmensetzung K1 oder eine hieraus in einfacher Weise erhältliche Katalysatorzusmmensetzung K2, K3 oder K4 eingesetzt wird. Bei diesen Zusammensetzungen handelt es sich um Reaktionsgemische, welche unmittelbar aus der Umsetzung einer Oxorheniumverbindung mit einem Organylierungsmittel resultieren (K1), wobei ein im Reaktionsgemisch enthaltener Überschuss an Organylierungsmittel mittels eines protischen Systems zerstört worden sein kann (K2) und Lösungsmittel und /oder anorganische Bestandteile in an sich bekannter einfacher Weise abgetrennt worden sein können (K3 bzw. K4). Die Abtrennung anorganischer Bestandteile kann zum Beispiel eine Filtration oder Extraktion umfassen, wobei eine Extraktion insbesondere dann vorteilhaft ist, wenn das Reaktionsgemisch nach der Umsetzung mit einem wässrigen Medium versetzt wurde, um überschüssiges Organylierungsmittel zu zerstören.

Ein wesentlicher Vorteil der Erfindung besteht somit darin, dass auf die bisher als erforderlich gehaltene Isolierung und Reinigung des als Katalysator wirksamen Organylrheniumoxids durch aufwändige Verfahren, wie Sublimation, Kristallisation oder Destillation unter Hochvakuum verzichtet werden kann. Überraschenderweise lässt sich als Katalysator für Oxidationsreaktionen eine Katalysatorzusammensetzung K1, K2, K3 oder K4 einsetzen, ohne dass gegenüber gereinigtem Organylrheniumoxid bei gleicher Re-Einsatzmenge eine Minderung der katalytischen Wirksamkeit in Kauf genommen werden muss. Es wird vermutet, dass das Organylierungsmittel eine mehr oder weniger stark ausgeprägte reduzierende Wirkung aufweist, die Katalysatorzusammensetzung mehr als ein einziges Organylrheniumoxid enthält. Offensichtlich werden Organylrheniumoxide, in welchen Rhenium in einer Oxidationsstufe kleiner 7 enthalten ist, durch das bei der Oxidation eingesetzte Oxidationsmittel in katalytisch wirksame Verbindungen der Oxidationsstufe +7 überführt oder/und in der Katalysatorzusammensetzung enthaltene Organylrheniumoxide der Re-Oxidationsstufe kleiner 7 sind selbst ausreichend katalytisch wirksam.

Zur Herstellung einer wirksamen Katalysatorzusammensetzung K1 oder einer daraus gewonnenen Katalysatorzusammensetzung K2, K3 bzw. K4 wird eine Oxorheniumverbindung der allgemeinen Formel ReOₘAₙ mit der bereits genannten Bedeutung für die Wertigkeit von Re, A, m und n eingesetzt. Eingesetzt werden beispielsweise die Rheniumoxide Re₂O₅, ReO₃ und Re₂O₇, wobei letzteres besonders bevorzugt wird. Im Falle der Oxide Re₂O₇ und Re₂O₅ handelt es sich bei der austretenden anionischen Gruppierung um das Perrhenation bzw. Rhenation. Im Falle des Rheniumoxids ReO₃ ist n gleich 0.

Bei weiteren Klassen geeigneter Rheniumverbindungen zur Herstellung der Katalysatorzusammensetzungen handelt es sich um Stoffe aus der Reihe HalReO₃, Hal₂ReO₂, HalReO₂, Hal₃ReO und Hal₃ ReO₂, wobei Hal für ein Halogen aus der Reihe Fluor, Chlor, Brom oder Jod steht, Chlor aber besonders bevorzugt ist.

Bei weiteren Klassen sehr gut geeigneter Oxorheniumverbindungen handelt es sich insbesondere um solche der allgemeinen Formel ReO₃-O-Acyl und ReO₂-O-Acyl, wobei die Klasse der 7-wertigen Rheniumverbindungen bevorzugt wird. Bei Acyl handelt es sich vorzugsweise um eine Gruppierung aus der Reihe R-C(O)-, Aryl-SO₂-, -P(O)(OR')₂, -Si(OR')₃, -P(O)R"₂ und -SiR"₃, worin R für gegebenenfalls fluoriertes Alkyl und R' und R" für Alkyl, Cycloalkyl, Arylalkyl oder Aryl stehen, die auch fluoriert sein können. Beispiele für diese Acylgruppen sind Acetyl, Trifluoracetyl, Benzoyl und Benzolsulfonyl, wobei der Phenylrest auch substituiert, wie fluoriert oder methyliert, sein kann, Trimethoxysilyl, Triethoxysilyl und Trimethylsilyl.

Die Oxorheniumverbindung wird mit einem Organylierungsmittel umgesetzt, wobei diese Umsetzung in einem aprotischen Lösungsmittel oder Lösungsmittelgemisch, insbesondere einem Ether, bei einer Temperatur im Bereich von etwa -70°C bis etwa +80°C, vorzugsweise im Bereich von -25 bis + 60 °C und insbesondere etwa 0°C bis 40°C durchgeführt wird.

Als Organylierungsmittel zur Herstellung der erfindungsgemäß zu verwendenden Katalysatorzusammensetzungen kommen Organylmetallverbindungen von Alkalimetallen, Magnesium, Kalzium, Aluminium und Zink in Betracht. Gemäß einer bevorzugten Ausführungsform wird als Organylierungsmittel ein Organylmagnesiumhalogenid verwendet. Beispiele geeigneter Organylierungsmittel sind LiR, NaR, KR, MgR₂, ZnR₂, AlR₃ und Al(O)R, insbesondere aber RMgHal, wobei Hal für ein Halogen, insbesondere Chlor steht. Der Organylrest R, bedeutet lineares, verzweigtes oder cyclisches Alkyl, insbesondere unverzweigtes C₁- bis C₄-Alkyl, besonders bevorzugt Methyl; R kann jedoch auch Benzyl oder Aryl bedeuten, wobei der Phenylrest oder Arylrest substituiert sein können.

Gemäß besonders bevorzugter Ausführungsformen wird als Organylierungsmittel eine Lithiumalkylverbindung oder ein Grignardreagenz, insbesondere ein Alkylmagnesiumhalogenid besonders bevorzugt Methylmagnesiumchlorid eingesetzt. Die Auswahl des Organylrestes wird der Fachmann derart wählen, dass die erhaltene Katalysatorzusammensetzung die höchste katalytische Wirksamkeit aufweist. Eine besonders hohe Wirksamkeit weisen Organylrheniumoxide oder Komplexe davon auf, welche eine oder mehrere Methylgruppen als Organylrest enthalten.

Wie zuvor ausgeführt wurde, wurden Oxorheniumverbindungen der allgemeinen Formel ReOₘAₙ mit siebenwertigem Re bisher nicht unter alleinigem Einsatz von Alkylmagnesiumhalogeniden, also ohne den Umweg über die Herstellung von Zinkalkylen, alkyliert. Die Verwendung von Alkylmagnesiumhalogeniden ist aber besonders vorteilhaft, weil sich diese Stoffklasse unter leicht handhabbaren Bedingungen erzeugen und anwenden lässt.

Zur Organylierung der Oxorheniumverbindung werden pro Mol derselben im Allgemeinen 1 bis 10 Organyläquivalente, vorzugsweise 1 bis 5 Äquivalente und insbesondere >1 bis 3 Organyläquivalente eingesetzt. Eine Einsatzmenge außerhalb des genannten Bereiches, beispielsweise 0,5 Organyläquivalente pro Mol Rheniumverbindung ist zwar möglich, bietet aber keine besonderen Vorteile.

Die Umsetzung der Oxorheniumverbindung mit dem Organylierungsmittel findet in der Regel in einem aprotischen Lösungsmittel statt. Geeignet sind Ether, wie Dialkylether und cyclische Ether, Ethergemische und Gemische aus Ethern mit aliphatischen und/oder aromatischen Kohlenwasserstoffen. Da Grignardreagenzien üblicherweise in einem ätherischen Lösungsmittel hergestellt werden, ist es besonders zweckmäßig, im gleichen Lösungsmittelsystem auch die Organylierung der Rheniumoxoverbindung durchzuführen. Es kann aber auch vorteilhaft sein, ein während der Grignardreagenz-Herstellung anwesendes ätherisches Lösungsmittel vor oder nach der Umsetzung mit der Oxorheniumverbindung gegen ein nicht-ätherisches aprotisches Lösungsmittel auszutauschen.

Sofern die Katalysatorzusammensetzung keinen Überschuss an Organylierungsmittel enthält oder ein geringer Überschuss die nachfolgende Oxidationsreaktion nicht nennenswert stört, kann eine derartige Katalysatorzusammensetzung, insbesondere wenn diese frei von ätherischen Lösungsmitteln ist, unmittelbar in der Oxidationsreaktion eingesetzt werden.

Während es sich bei der Zusammensetzung K1 im Allgemeinen um ein lösungsmittelhaltiges System handelt, handelt es sich bei dem Katalysatorsystem K3 um ein von Lösungsmitteln zumindest teilweise befreites und/oder von anorganischen Bestandteilen mindestens teilweise befreites Katalysatorsystem. Die Abtrennung von Lösungsmitteln erfolgt in an sich bekannter Weise, üblicherweise durch Abdestillieren unter vermindertem Druck. Auch die Abtrennung von anwesenden anorganischen Bestandteilen in einem lösungsmitelhaltigen Katalysatorsystem K1 kann mittels üblicher Methoden zur Fest-Flüssig-Trennung bewirkt werden, darunter Filtrationsmethoden, oder durch Auflösen der in einem in K1 enthaltenen organischen Lösungsmittelsystem unlöslichen anorganischen Bestandteile in einem zugegebenen wässrigen System mit nachfolgender Phasentrennung und bei Bedarf Extraktion mit einem wasserunlöslichen oder wenig wasserlöslichen organischen Lösungsmittel.

Sofern zur Organylierung der Rheniumverbindung das Organylierungsmittel im Überschuss eingesetzt wird, empfiehlt es sich, diesen Überschuss nach der Umsetzung durch Zugabe eines protischen Mittels zu zerstören und bei Bedarf auch die dabei ausfallenden Feststoffe von dem gebildeten Organylrheniumoxid abzutrennen. Zweckmäßigerweise wird das protische Mittel in mindestens stöchiometrischer Menge eingesetzt. Beispiele geeigneter protischer Mittel sind Lösungsmittel aus der Reihe Wasser, Alkohole, Carbonsäuren, Kieselsäuren, Borsäuren und Mineralsäuren. Durch die Zersetzung des Überschusses an Organylierungsmittel, welche vor oder nach Abtrennung von Lösungsmitteln und/oder unlöslichen Bestandteilen erfolgen kann, wird die Katalysatorzusammensetzung K2 gebildet. Anstelle eines flüssigen Mittels zur Zersetzung eines Überschusses an Organylierungsmittel können auch Feststoffe verwendet werden, welche entweder Hydroxylfunktionen enthalten, Beispiele sind Kieselsäuren und Aluminiumoxide, und/oder an ihrer Oberfläche adsorbiertes Wasser enthalten. Ein Vorteil derartiger fester Zersetzungsmittel ist es, dass diese leicht aus der Katalysatorzusammensetzung K2 abgetrennt werden können, wobei eine Katalysatorzusammensetzung K4 entsteht. Bei Bedarf kann aus der Zusammensetzung K4 auch das darin enthaltene Lösungsmittel ganz oder teilweise entfernt oder gegen ein anderes Lösungsmittel ausgetauscht worden sein. Die Entfernung des Lösungsmittels ist insbesondere dann notwendig, wenn die Oxidationsreaktion in einem anderem Lösungsmittelsystem als demjenigen aus der Herstellung der enthaltenden Katalysatorzusammensetzung durchgeführt werden soll.

Die Herstellung der mindestens eine Organylrheniumverbindung enthaltenden Katalysatorzusammensetzung kann in Gegenwart einer als Ligand für das Organylrheniumoxid dienenden Lewis-basischen Verbindung durchgeführt werden; in diesem Fall ist d in der allgemeinen Formel RₐRe_{b}O_{c}L_{d} ungleich 0. Geeignete Liganden sind aliphatische und aromatische Amine und insbesondere N-heteroaromatische Verbindungen. Es werden zweckmäßigerweise solche Liganden eingesetzt, welche unter den Bedingungen der nachfolgenden Oxidationsreaktion oder sonstigen Reaktionen in Gegenwart eines Katalysators der Formel RₐRe_{b}O_{c}L_{d} nicht selbst angegriffen werden und damit die Aktivität des Katalysators mindern oder zu unerwünschten Nebenprodukten führen.

Das erfindungsgemäße Verfahren unter Verwendung einer erfindungsgemäßen Katalysatorzusammensetzung lässt sich zur Oxidation sehr unterschiedlicher organischer Substrate verwenden. Derartige Oxidationsreaktionen sind im Stand der Technik bereits beschrieben worden, wobei jedoch die als Katalysator verwendete Organyloxorheniumverbindung, wie Methyltrioxorhenium (MTO) in reiner Form eingesetzt wurde. Beispiele für oxidationsfähige Substrate sind Olefine, welche je nach den angewandten Temperatur- und Lösungsmittelbedingungen epoxidiert und/oder zu vicinalen Diolen hydroxyliert werden können. Das Prinzip und die Reaktionsbedingungen sind beispielhaft der EP-B 0 380 085 zu entnehmen. Im Verfahren dieses Dokuments wurden auch Komplexe von Organylrheniumoxiden mit Lewis-basischen Liganden L in gereinigter Form als Katalysator für die Epoxidation oder Hydroxylierung von Olefinen eingesetzt. Im erfindungsgemäßen Verfahren ist es dagegen möglich, eine Katalysatorzusammensetzung gemäß K1, K2, K3 oder K4, wobei diese bereits eine Lewis-basische Verbindung in komplex gebundener Form enthalten kann oder ein solcher Komplex erst in situ durch die Zugabe eines Liganden L bei der Anwendung der Katalysatorzusammensetzung gebildet wird, ohne Isolierung eines darin enthaltenen katalytisch wirksamen Organyloxorheniumoxids oder Komplexes hiervon als Katalysator für die erfindungsgemäßen Oxidationsreaktionen zu verwenden. Gemäß einer bevorzugten Ausführungsform werden in das Reaktionssystem zur Durchführung der Oxidation sowohl eine erfindungsgemäß zu verwendende Katalysatorzusammensetzung als auch eine Lewis-basische Verbindung gegeben, wobei sich der katalytisch wirksame Komplex in-situ bildet.

Bei den Lewis-basischen Verbindungen handelt es sich vorzugsweise um Stickstoffbasen, insbesondere Stickstoffheterozyklen, wie insbesondere Pyridin, Pyrazol oder Dipyridin, wobei diese Heterozyklen auch substituiert sein können. Weitere geeignete Stickstoffbasen sind 1-Azabicyclo[2,2,2]octan und diverse aromatische Amine, wobei sich die Eignung der letzteren teilweise danach richtet, welche Reaktionsbedingungen gewünscht werden, da diese aromatischen Amine je nach den gewählten Reaktionsbedingungen selbst oxidiert werden können.

Gemäß einer speziellen Ausführungsform der Erfindung werden sekundäre Amine zu disubstituierten Hydroxylaminen oxidiert. Einsetzbar sind aliphatische sekundäre Amine, wobei die am Stickstoff gebundenen Alkylreste auch einen oder mehrere Substituenten aufweisen können. Gemäß einer besonders bevorzugten Ausführungsform handelt es sich bei dem zu hydroxylierenden Amin um ein Bis(hydroxyalkyl)amin, beispielsweise Bis(2-methoxyethyl)amin, Bis(2-ethoxyethyl)amin, Bis(2-n- oder iso-propoxyethyl)amin und Bis(2-n-butoxyethyl)amin sowie die analogen Bis(alkoxypropyl)- und Bis(alkoxybutyl)amine. Auch sekundäre Amine mit einem aliphatischen und einem aromatischen Rest, wie N-Methoxyethylanilin, lassen sich gut in die entsprechenden Hydroxylamine überführen.

Eine weitere Klasse erfindungsgemäß zugänglicher Oxidationsreaktionen basiert auf der Baeyer-Villiger-Reaktion, wobei cyclische Ketone, wie Cyclopentanon oder Cyclohexanon, die ihrerseits substituiert sein können, zu den entsprechenden Lactonen oxidiert werden.

Unter Verwendung einer erfindungsgemäßen Katalysatorzusammensetzung lassen sich auch Aromaten, insbesondere mehrkernige Aromaten, wie Naphthalin, Alkylnaphthaline, Anthracene und Alkylantracene in die entsprechenden Chinonkörper überführen.

Weitere mit einer Peroxoverbindung, wie insbesondere Wasserstoffperoxid in Gegenwart einer erfindungsgemäßen Katalysatorzusammensetzung oxidierbare Substrate sind: Alkine, woraus Carbonsäuren oder α-Diketone entstehen; organische Sulfide, welche in die Sulfoxide oder Sulfone überführt werden können; die Oxidation von aromatischen Aminen zu Nitrosoaminen, sowie aromatischen N,N-Dialkylaminen zu den entsprechenden N-Oxiden.

Die erfindungsgemäßen Katalysatorzusammensetzungen eignen sich wie die isolierten bekannten Organylrheniumoxide außer als Katalysator für Oxidationsreaktionen auch als Katalysator für die Aldehydolefinierung, die Olefin-Methathese sowie für Diels-Alder-Reaktionen.

Die erfindungsgemäßen Oxidationsreaktionen werden in an sich bekannter Weise in einem Lösungsmittelsystem durchgeführt, und das Lösungsmittelsystem kann ein- oder mehrphasig sein und auch Wasser enthalten.

Zweckmäßigerweise wird allerdings der Wassergehalt niedrig gehalten, um eine hydrolytische Zersetzung des katalytisch wirksamen Systems zu vermeiden oder gering zu halten.

Oxidationsmittel im erfindungsgemäßen Verfahren ist eine Peroxoverbindung, insbesondere eine Quelle für Wasserstoffperoxid, welche unter den Reaktionsbedingungen Wasserstoffperoxid freizusetzen vermag, oder unmittelbar Wasserstoffperoxid. Wasserstoffperoxid wird bevorzugt als wässrige Lösung eingesetzt, insbesondere als wässrige Lösung mit einem H₂O₂-Gehalt von 35 bis 80 Gew.-%. Unter den Quellen für Wasserstoffperoxid sind Persalze zu nennen, wie Natriumperboratmonohydrat, Natriumperborattetrahydrat, Natriumpercarbonat, Persulfate und Persilikate. Bei Verwendung von basischen Persalzen muss ggf. vor der Kontaktierung mit der Katalysatorzusammensetzung der pH-Wert abgesenkt werden. Einsetzbar sind auch H₂O₂-Addukte, etwa solche an Harnstoff (=Percarbamid) oder an Kieselsäuren. Üblicherweise wird das Oxidationsmittel in mindestens stöchiometrisch erforderlicher Menge eingesetzt, jedoch hat sich ein gewisser Überschuss in vielen Fällen als vorteilhaft herausgestellt.

Weiterer Gegenstand der Erfindung ist eine Katalysatorzusammensetzung, insbesondere geeignet zur Durchführung von Oxidationsreaktionen, enthaltend mindestens ein Organylrheniumoxid der Formel RₐRe_{b}O_{c}L_{d}, worin R für einen Organylrest, a für eine ganze Zahl von 1 bis 6, b für eine ganze Zahl von 1 bis 4, c für eine ganze Zahl von 1 bis 12, d für eine ganze Zahl von 0 bis 4, L für einen Lewis-basischen Ligand stehen und Re fünf- bis siebenwertig ist, und vorliegend als Katalysatorzusammensetzung K1 oder einer daraus gewonnenen Katalysatorzusammensetzung K2 bis K4, dadurch gekennzeichnet ist, dass sie erhalten worden ist durch ein Verfahren, umfassend Organylierung einer Oxorheniumverbindung der allgemeinen Formel ReOₘAₙ , wobei Re fünf- bis siebenwertig ist, A für eine anionische Abgangsgruppe, m für eine ganze Zahl 1, 2 oder 3 und n für eine ganze Zahl 0, 1, 2 oder 3 stehen, mit einer Grignardverbindung der Formel RMgCl oder RZnHal, worin R für einen Organylrest, steht, und wobei pro Mol ReOₘAₙ 0,5 bis 10 Mol der Grignardverbindung eingesetzt werden, in einem aprotischen Lösungsmittelsystem in An- oder Abwesenheit einer Lewis-basischen Ligandenverbindung L unter Erhalt einer Katalysatorzusammensetzung K1, Zersetzung überschüssiger Grignardverbindung in der Katalysatorzusammensetzung K1 durch Zugabe eines protischen Mittels unter Erhalt einer Katalysatorzusammensetzung K2, mindestens teilweises Entfernen von Lösungsmitteln und/oder anorganischen Bestandteilen aus K1 beziehungsweise K2 unter Erhalt einer Katalysatorzusammensetzung K3 beziehungsweise K4, wobei die Herstellung von K1 bis K4 eine Sublimation, Kristallisation oder Destillation der darin enthaltenen Organylrheniumoxide der Formel RₐRe_{b}O_{c}L_{d} ausschließt und wobei bei der Herstellung der Katalysatorzusammensetzung ein Organylmagnesiumchlorid eingesetzt wurde, wobei Organyl ausgewählt ist aus der Reihe Methyl, Ethyl, n-Propyl, Isopropyl, n-Butyl, sek.-Butyl und Allyl.

Die Unteransprüche richten sich auf bevorzugte Ausführungsformen der Katalysatorzusammensetzung, wobei bezüglich der Reaktionspartner zur Herstellung der Katalysatorzusammensetzung K1 bis K4 auf die vorstehenden Ausführungen Bezug genommen wird.

Die erfindungsgemäße unter Verwendung einer GrignardVerbindung hergestellte Katalysatorzusammensetzung in der Ausführungsform K1 bis K4 enthält in der Regel mehrere Organyloxorheniumverbindungen. Es ist ein Vorteil der erfindungsgemäßen Katalysatorzusammensetzung, dass die darin enthaltenen Organylrheniumoxide nicht aus dem Stoffgemisch isoliert und gereinigt werden müssen, um wirksam als Katalysator verwendet werden zu können. Ein weiterer Vorteil liegt in der leichten Zugänglichkeit und problemlosen Handhabung der Grignardreagenzien.

Die Erfindung wird anhand der nachfolgenden Beispiele weiter verdeutlicht.

### Beispiele 1.1 bis 1.12

Herstellung einer Katalysatorzusammensetzung und Verwendung derselben zur Epoxidation von Cycloocten.

Als Oxorheniumverbindung wurde eine Lösung von Re₂O₇ in Tetrahydrofuran eingesetzt (c = 0,04 mmol Re₂O₇/ml)(=Lösung A). Als Organylierungsmittel wurde Methylmagnesiumchlorid in Tetrahydrofuran (c = 3,0 mol/l)(=Lösung B) eingesetzt.

### Herstellung der Katalysatorzusammensetzung

Je 0,5 ml (R1 - R4) bzw. 2,5 ml (R5 - R12) der Lösung A wurden in inertisierte Glasreaktoren dosiert. In die Reaktoren R4 bis R6 wurden je 16 µl und in die Reaktoren R10 bis R12 je 81 µl Pyridin gegeben. Nach Abkühlen auf 0°C wurde die Lösung B zugegeben. Das Molverhältnis Methylmagnesiumchlorid zu Rheniumheptoxid folgt aus Tabelle 1. Nach 2h Reaktionszeit wurde auf 25°C erwärmt. Zwecks Zerstörung überschüssigen/nicht umgesetzten Methylmagnesiumchlorids wurde jeder Reaktor mit 4 ml 10 gew.-%-iger wässriger NH₄Cl-Lösung versetzt. Nach 5 min wurde jeder Ansatz mit 4 ml CH₂Cl₂ extrahiert. Bei der abgetrennten organischen Phase handelt es sich um eine von anorganischen Bestandteilen befreite Katalysatorzusammensetzung K4. Soweit anwesend fungierte das zugesetzte Pyridin zumindest teilweise als Ligand L.

Die jeweilige organische Phase wurde in 12 neue Reaktoren R1 bis R12 überführt, dann wurden im Vakuum bei 25°C die Lösungsmittel CH₂Cl₂ und Tetrahydrofuran entfernt. Erhalten wurden von anorganischen Bestandteilen und Lösungsmitteln im Wesentlichen befreite Katalysatorzusammensetzungen K4.

### Epoxidation

Die Rückstände in R1 bis R12 wurden in jeweils 4 ml CH₂Cl₂ aufgenommen, mit jeweils 1,8 ml H₂O₂-Lösung (35 Gew.-%) und 80 µl Pyridin und 160 µl Tetradecan (= innerer Standard für die GC-Auswertung) versetzt. Unter mäßiger Kühlung ( ca. 19°C) wurde jeweils 1,0 ml Cycloocten zudosiert. Nach 20 h Reaktionszeit wurden Proben entnommen, mit 1 ml Methylenchlorid und einer Spur MnO₂ versetzt und nach Zersetzung von restlichem H₂O₂ die organische Phase über Kieselgel filtriert und mittels GC analysiert (Säule: CP-SIL 13CB WCOT Fused Silica).

Tabelle 1 zeigt die molaren Einsatzmengen bei der Herstellung der Katalysatorzusammensetzungen, die Katalysatormenge bei der Epoxidation und die bei der Epoxidation erhaltenen Ausbeuten an Cyclooctenoxid:

**Tabelle 1**

| Reaktor- Nr. | Methylierung | | | Ausbeute Cyclooctenoxid |
|---|---|---|---|---|
| | Re₂O₇¹⁾ | Pyridin | CH₃MX | |
| R | mol-%_{Re}²⁾ | mol/mol Re₂O₇ | mol/mol Re₂O₇ | (%) |
| 1 | 0,5 | | 1 | 65,4 |
| 2 | 0,5 | | 3 | 92,4 |
| 3 | 0,5 | | 5 | 96,0 |
| 4 | 0,5 | 10 | 1 | 72,7 |
| 5 | 0,5 | 10 | 3 | 90,4 |
| 6 | 0,5 | 10 | 5 | 94,7 |
| 7 | 2,6 | | 1 | 95,0 |
| 8 | 2,6 | | 3 | 96,2 |
| 9 | 2,6 | | 5 | 95,3 |
| 10 | 2,6 | 10 | 1 | 97,1 |
| 11 | 2,6 | 10 | 3 | 97,0 |
| 12 | 2,6 | 10 | 5 | 95,9 |

| | | | | |
|---|---|---|---|---|
| ¹⁾ In der Methylierung wurde Re₂O₇ eingesetzt ²⁾ Die Angabe mol-% Re ist die Einsatzmenge Re, bezogen auf das zu epoxidierende Olefin | | | | |

### Beispiele 2.1 bis 2.12

Analog den Beispielen 1.1 bis 1.12 wurden die Beispiele 2.1 bis 2.12 durchgeführt, jedoch wurde als Organylierungsmittel ein Methyllithium-Lithiumbromid-Komplex in Diethylether (c = 1,5 mol/l) eingesetzt. Tabelle 2 zeigt die molaren Einsatzmengen bei der Herstellung der Katalysatorzusammensetzungen und die bei der Epoxidation erhaltenen Ausbeuten an Cyclooctenoxid:

**Tabelle 2**

| Reaktor- Nr | Methylierung | | | Ausbeute Cyclooctenoxid (%) |
|---|---|---|---|---|
| | Re₂O₇¹⁾ | Pyridin | LiCH₃ | |
| | mol-%_{Re}²⁾ | mol/mol Re₂O₇ | mol/mol Re₂O₇ | |
| 1 | 0,5 | | 1 | 96,3 |
| 2 | 0,5 | | 3 | *) |
| 3 | 0,5 | | 5 | *) |
| 4 | 0,5 | 10 | 1 | 76,9 |
| 5 | 0,5 | 10 | 3 | *) |
| 6 | 0,5 | 10 | 5 | 82,8 |
| 7 | 2,6 | | 1 | 94,1 |
| 8 | 2,6 | | 3 | 92,6 |
| 9 | 2,6 | | 5 | 95,3 |
| 10 | 2,6 | 10 | 1 | 92,3 |
| 11 | 2,6 | 10 | 3 | 96, |
| 12 | 2,6 | 10 | 5 | 94,3 |

| | | | | |
|---|---|---|---|---|
| ¹⁾ In der Methylierung wurde Re₂O₇ eingesetzt ²⁾ Die Angabe mol-% Re ist die Einsatzmenge Re, bezogen auf das zu epoxidierende Olefin *) Versuchsfehler | | | | |

### Vergleichsbeispiele VB1 bis VB6

Die Epoxidation von Cycloocten erfolgte in analoger Weise wie in den erfindungsgemäßen Beispielen, eingesetzt wurden jedoch anstelle einer erfindungsgemäßen Katalysatorzusammensetzung folgende Stoffe bzw. Reaktionsgemische.
VB1: MTO (= reines Methyltrioxorheniumoxid)
VB2: Äquimolares Gemisch aus MTO und KReO₄
VB3: Re₂O₇
VB4: KReO₄
VB5 und VB6: Reaktionsgemisch, erhalten durch Kontaktieren von KReO₄ mit 1 bzw. 5 Äquivalenten CH₃MgCl pro Äquivalent Re, Behandeln mit wässriger NH₄Cl-Lösung, Extraktion mit CH₂Cl₂, Phasentrennung und Verwendung der organischen Phase, enthaltend "KReO₄ methyliert".

Tabelle 3 zeigt die Ergebnisse

**Tabelle 3**

| VB-Nr. | Re-Katalysator | Mol-% Re, bez. auf Olefin | CH₃MgCl mol/mol Re | Ausbeute Cyclooctenoxid (%) |
|---|---|---|---|---|
| 1 | MTO | 0,5 | - | 94, 8 |
| 2 | MTO/KReO₄ (1:1) | 0,5 | - | 89,1 |
| 3 | Re₂O₇ | 0,5 | - | 0,2 |
| 4 | KReO₄ | 0,5 | - | 0,2 |
| 5 | "KReO₄ methyliert" | 2 , 5 | 1 | 0,2 |
| 6 | "KReO₄ methyliert" | 2 , 5 | 5 | 0 |

Beim Vergleich des Vergleichsbeispiels VB1 mit den erfindungsgemäßen Beispielen 1.1 bis 1.3 sowie alternativ 2.1 ist erkennbar, dass die katalytische Aktivität im Wesentlichen gleich ist. Durch Absenken der Einsatzmengen an MTO durch Substitution der Hälfte durch die Re-äquivalente Menge KReO₄ bei Aufrechterhaltung der molaren Einsatzmenge an Re-Verbindungen bei der Epoxidation sinkt die Epoxidationsausbeute etwas ab (VB2). Dieser Ausbeuteabfall war im Hinblick auf das Ergebnis des Vergleichsbeispiels VB4 zu erwarten, da sich KReO₄ als katalytisch inaktiv erwies. Wie aus VB5 und VB6 folgt, lässt sich KReO₄ unter den erfindungsgemäßen Alkylierungsbedingungen offensichtlich nicht alkylieren, denn ein so gewonnenes Reaktionsgemisch ist katalytisch inaktiv.

Völlig überraschend ist der Vergleich der Ergebnisse der erfindungsgemäßen Beispiele 1.2 und 1.3 oder 2.1 mit dem Vergleichsbeispiel VB2, weil sich die katalytische Wirksamkeit der erfindungsgemäßen Katalysatorzusammensetzungen unter den Bedingungen der Beispiele sogar als höher erwies, als jene des Gemischs aus reinem MTO und KReO₄ (molares Verhältnis 1:1). Nach dem Stand der Technik (siehe Seiten 3210 - 3211 des zitierten Review-Artikels) entstehen bei der Methylierung von Re₂O₇ mit einem Alkylierungsmittel theoretisch MTO und Perrhenat in äquivalenter Menge; in der Realität liegt der Gehalt an MTO je nach den gewählten Bedingungen durch Folgereaktionen, wie Reduktionsreaktionen, deutlich niedriger; bei der als erforderlich gehaltenen Isolierung des MTO aus dem Reaktionsgemisch lassen sich zudem Verluste an MTO nicht vermeiden. Bei der erfindungsgemäßen Katalysatorzusammensetzung entfalten demgegenüber offensichtlich auch andere bei der Alkylierung von Re₂O₇ erzeugte Alkylrheniumoxide eine katalytische Wirkung und zudem werden durch die direkte Verwendung der Katalysatorzusammensetzung Verluste an MTO und/oder anderen katalytisch wirksamen Alkylrheniumoxiden vermieden.

### Beispiele 3.1 bis 3.6

### Epoxidation von 1-Hexen

### a) Herstellung der Katalysatorzusammensetzungen für die Beispiele 3.1 bis 3.6 und 4.1 bis 4.6

Eingesetzt wurden folgende Lösungen von Re₂O₇ in Tetrahydrofuran (THF):
Variante V1: 813,8 mg Re₂O₇ in 42 ml THF (= 0,040 mol/l)
Variante V2: 542,5 mg Re₂O₇ in 42 ml THF (= 0,0267 mol/l)
Variante V3: 406,9 mg Re₂O₇ in 42 ml THF (= 0,020 mol/l)
Variante V4: 203,3 mg Re₂O₇ in 42 ml THF (= 0,010 mol/l)

Die Herstellung der Katalysatorlösung erfolgt unter Sauerstoff- und Feuchteausschluss. In einem 25 ml Zweihalskolben mit Magnetrührer und Innenthermometer werden 2,5 ml der Lösung V1, V2, V3 oder V4 vorgelegt und auf 0°C gekühlt. Hierzu wird vorsichtig eine 3-molare Lösung von Methylmagnesiumchlorid in Tetrahydrofuran zugegeben, und zwar pro mol Re₂O₇ 3 mol (A), 4,5 mol (B), 6 mol (C) oder 12 mol (D) CH₃MgCl.

Es erfolgt ein sofortiger Farbumschlag der Lösung nach braun. Die Lösung wird insgesamt 2h bei 0°C im Dunkeln nachgerührt und nach Entfernung des Eisbades noch 5 min gerührt und anschließend auf Raumtemperatur erwärmen gelassen. Zur Hydrolyse werden 4 ml einer 10%igen Ammoniumchloridlösung zugegeben und die Mischung 5 min. gerührt. Anschließend wird eine Extraktion mit 4 ml Dichlormethan durchgeführt. Die abgetrennte untere, organische Phase, insgesamt 6,5 ml, wird als Katalysatorlösung zur Oxidation eingesetzt. In den Tabellen 4 und 5 wird die Einsatzmenge (ml) an diesen Katalysatorlösungen angegeben.

### Oxidation von 1-Hexen

In einem 25 ml Dreihalskolben wurden bei Raumtemperatur 1ml (7,64 mmol) 1-Hexen und 80 µl (12,97 mol%) Pyridin vorgelegt und, sofern nicht anders angegeben, mit 6,5 ml der in der Tabelle 4 angegebenen Katalysatorlösung versetzt. Zu der Lösung wurden unter Rühren langsam 0,9 ml (18,56 mmol, also 2,43 mol H₂O₂ pro mol 1-Hexen) wässriges Wasserstoffperoxid (70 Gew.-%) so zugegeben, dass die Temperatur nicht über 30°C steigt. Die GC-Rohausbeute an Hexenoxid nach 26h Reaktionszeit folgt aus der letzten Spalte der Tabelle 4.

### Zu Beispiel 3.5:

Zu 6,5 ml Katalysatorlösung V1A wurden unter Rühren bei Raumtemperatur langsam 0,9 ml (18,56 mmol) wässriges Wasserstoffperoxid (70 Gew.-%) zugegeben, wobei eine Emulsion entsteht. In einem 25 ml Dreihalskolben wurden bei Raumtemperatur 1 ml (7,64 mmol) 1-Hexen und 80 µl (12,97 mol%) Pyridin vorgelegt und die H₂O₂ und Katalysator enthaltende Emulsion langsam so zugegeben, dass die Innentemperatur nicht über 30°C stieg. Die GC-Rohausbeute nach 26h Reaktionszeit beträgt 93,2% an Hexenoxid.

### Zu Beispiel 3.6:

Eingesetzt wurde die aus V1C gebildete, vom Lösungsmittel befreite Katalysatorzusammensetzung. Die Umsetzung erfolgte somit in Abwesenheit eines Lösungsmittels.

### Zu Beispiel 3.7:

Die Oxidation erfolgte bei einem Molverhältnis H₂O₂/Olefin von 1,25, also einem wesentlich geringerem Verhältnis als in den Beispielen 3.1 bis 3.6. Wie die Tabelle zeigt, wird jedoch eine wesentlich höhere Ausbeute erzielt als im Beispiel 3.1 mit der gleichen Katalysatorart und -menge aber höherem Molverhältnis H₂O₂/Olefin.

**Tabelle 4:**

| Bsp- Nr. | Katalysatorlösung | | Re₂O₇ mol%^{#} | CH₃MgCl äq. auf Re₂O₇ | CH₂Cl₂^{##} ml | Hexenoxid (GC%) |
|---|---|---|---|---|---|---|
| | in ml | Variante | | | | |
| 3.1 | 6,5 | V1A | 1,30 | 3 | - | 52,5 |
| 3.2 | 6,5 | V1A | 1,30 | 3 | 4 | 82,2 |
| 3.3 | 6,5 | V1A | 0,651 | 6 | - | 78,2 |
| 3.4 | 6,5 | V1C | 1,30 | 6 | 4 | 87,2 |
| 3.5 | 6,5* | V1A | 1,30 | 6 | - | 93,2 |
| 3.6 | 6,5** | V1C | 1,30 | 6 | - | 82,6 |
| 3.7 | 6,5*** | V1A | 1,30 | 3 | - | 78,8 |

| | | | | | | |
|---|---|---|---|---|---|---|
| *), **) und ***) siehe Anmerkungen vor der Tabelle # mol% Re₂O₇ bezogen auf 1-Hexen ## zusätzliche Zugabe vor der Umsetzung | | | | | | |

### Beispiele 4.1 bis 4.6

### Oxidation von N,N-Bis-(2-methoxyethyl)-amin

In einem 25 ml Dreihalskolben wurden 5ml (3,71g; 34,16 mmol) N,N-Bis-(2-methoxyethyl)-amin, 15ml CH₂Cl₂ und 32 µl (1,16 mol%) Pyridin vorgelegt und mit in Tabelle 5 angegebener Menge und Art der Katalysatorlösung versetzt. Die Lösung wurde nun auf -15°C gekühlt und langsam wurden unter Rühren insgesamt 1,91 ml (1,3 äq; 44,41 mmol) wässriges Wasserstoffperoxid (70 Gew.-%) so zugegeben, dass die Innentemperatur nicht über 5°C anstieg. Insbesondere in der Anfangsphase ist die Reaktion sehr exotherm. Nach beendeter Zugabe wurde bei ca. -15°C noch 4h nachgerührt. Die Lösung ist dann zweiphasig. Die Tabelle zeigt die durch GC-Auswertung ermittelten Ausbeuten an Bis-N,N-(2-methoxyethyl)-hydroxylamin (DMEHA), nicht umgesetztem Edukt und durch Überoxidation gebildetem Nitron.

**Tabelle 5**

| Bsp-Nr. | Katalysatorlösung | | Re₂O₇ (mol%) | CH₃MgCl äq. auf Re₂O₇ | CH₂Cl₂ (ml) | Edukt (GC%) | DMEHA (GC%) | Nitron (GC%) |
|---|---|---|---|---|---|---|---|---|
| | V in ml | Variante | | | | | | |
| 4.1 | 2,22 | V1A | 0,10 | 3 | 15 | 6,39 | 82,5 | 11,1 |
| 4.2 | 2,22 | V1C | 0,10 | 6 | 15 | 0,4 | 83,3 | 16,0 |
| 4.3 | 2,22 | V2B | 0,066 | 4,5 | 15 | 1,8 | 83,6 | 13,8 |
| 4.4 | 1,11 | V1C | 0,05 | 6 | 15 | 4,39 | 81,86 | 13,75 |
| 4.5 | 2,22 | V3C | 0,05 | 6 | 15 | 1,2 | 82,2 | 11,5 |
| 4.6 | 2,22 | V4C | 0,025 | 6 | 15 | 6,4 | 79,5 | 12,9 |

| | | | | | | | | |
|---|---|---|---|---|---|---|---|---|
| *GC% nach 4h Nachrührzeit bei -15°C | | | | | | | | |

### Vergleichsbeispiel VB7

Zur Aminoxidation wurde mit 0,05 mol% MTO katalysiert. Erhalten wurden 82 % DMEHA, 1,5 % Edukt und 14 % Nitron.

## Patentansprüche

1. Verfahren zur Oxidation organischer Substrate,
umfassend Umsetzung des organischen Substrats in einem Lösungsmittelsystem mit einer Peroxoverbindung, insbesondere Wasserstoffperoxid, in Gegenwart eines Organylrheniumoxids der Formel RₐRe_{b}O_{c}L_{d}, worin R ein Organylrest, a eine ganze Zahl von 1 bis 6, b eine ganze Zahl von 1 bis 4, c eine ganze Zahl von 1 bis 12, d eine ganze Zahl von 0 bis 4, L ein Lewis-basischer Ligand und Re fünf- bis siebenwertig ist, als Katalysator, **dadurch gekennzeichnet, dass** man eine Katalysatorzusammensetzung K1 oder eine daraus gewonnene Katalysatorzusammensetzung K2, K3 oder K4 einsetzt, wobei die Herstellung von K1 die Umsetzung einer Oxorheniumverbindung der allgemeinen Formel ReOₘAₙ, worin Re fünf- bis siebenwertig ist, A für eine anionische Abgangsgruppe, m für die Zahl 1, 2 oder 3 und n für die Zahl 0, 1, 2 oder 3 stehen, mit 1 bis 10 Organyläquivalenten pro Mol ReOₘAₙ eines Organylierungsmittels eines Elements aus der Reihe Li, Na, K, Mg, Al und Zn in einem aprotischen Lösungsmittel in An- oder Abwesenheit eines Lewis-basischen Liganden umfasst, die Herstellung von K2 die Zugabe eines protischen Mittels zu einer überschüssiges Organylierungsmittel enthaltenden Zusammensetzung K1 und die Herstellung von K3 beziehungsweise K4 mindestens teilweises Entfernen von Lösungsmitteln und/oder anorganischen Bestandteilen aus K1 beziehungsweise K2 umfasst, die Herstellung von K1 bis K4 aber eine Sublimation, Kristallisation oder Destillation des oder der darin enthaltenen Organylrheniumoxide der Formel RₐRe_{b}O_{c}L_{d} ausschließt.

2. Verfahren nach Anspruch 1, **dadurch gekennzeichnet, dass** man Olefine als Substrat epoxidiert oder zu Diolen hydroxyliert.

3. Verfahren nach Anspruch 1, **dadurch gekennzeichnet, dass** man Amine als Substrat oxidiert, insbesondere sekundäre Amine zu disubstituierten Hydroxylaminen oxidiert.

4. Verfahren nach Anspruch 1, **dadurch gekennzeichnet, dass** man cyclische Ketone als Substrat einsetzt und diese nach Baeyer-Villiger zu Lactonen oxidiert.

5. Verfahren nach Anspruch 1, **dadurch gekennzeichnet, dass** man Aromaten, insbesondere mehrkernige Aromaten als Substrat einsetzt und diese zu Chinonen oxidiert.

6. Verfahren nach einem der Ansprüche 1 bis 5, **dadurch gekennzeichnet, dass** man als Oxorheniumverbindung eine Verbindung aus der Reihe ReO₃ , Re₂O₇ , Hal-Re0₃, Hal₂ReO₂, ReO₃-O-Acyl einsetzt, wobei Hal für F, Cl, Br oder J und Acyl für eine Gruppe aus der Reihe R-C(O)-, Aryl-SO₂-, -P(O)(OR')₂, -Si(OR')₃, -P(O)R''₂ und -SiR''₃, worin R für gegebenenfalls fluoriertes Alkyl und R' und R" für Alkyl, Cycloalkyl, Arylalkyl oder Aryl stehen, die auch fluoriert sein können.

7. Verfahren nach einem der Ansprüche 1 bis 6, **dadurch gekennzeichnet, dass** man als Organylierungsmittel eine Verbindung aus der Reihe LiR, NaR, KR, MgR₂, RMgHal, ZnR₂, AlR₃ und Al(O)R einsetzt, wobei R für lineares, verzweigtes oder cyclisches Alkyl, insbesondere unverzweigtes C₁-C₄-Alkyl, Benzyl oder Aryl, das alkylsubstituiert sein kann, steht.

8. Verfahren nach einem der Ansprüche 1 bis 6, **dadurch gekennzeichnet, dass** man als Organylierungsmittel eine Verbindung aus der Reihe der Lithiumalkylverbindungen und der Grignard-Reagenzien einsetzt, insbesondere ein Alkylmagnesiumhalogenid, und besonders bevorzugt Methylmagnesiumchlorid.

9. Verfahren nach einem der Ansprüche 1 bis 8, **dadurch gekennzeichnet, dass** man pro Mol Oxorheniumverbindung 1 bis 5, insbesondere >1 bis 3 Organyläquivalente einsetzt.

10. Verfahren nach einem der Ansprüche 1 bis 9, **dadurch gekennzeichnet, dass** man die Organylierung der Oxorheniumverbindung und/oder die Oxidationsreaktion in Gegenwart einer Stickstoffbase als Ligand L , die mit in der Katalysatorzusammensetzung enthaltenen Organylrheniumoxiden Komplexe der Formel RₐRe_{b}O_{c}L_{d} bilden kann, durchführt.

11. Verfahren nach einem der Ansprüche 1 bis 10, **dadurch gekennzeichnet, dass** man im Reaktionsgemisch K1 enthaltene überschüssige Organylierungsmittel durch Zugabe einer mindestens stöchiometrischen Menge eines Lösungsmittels aus der Reihe Wasser, Alkohole, Carbonsäuren, Kieselsäuren, Borsäuren und Mineralsäuren zersetzt, wobei die Katalysatorzusammensetzung K2 gebildet wird.

12. Verfahren nach einem der Ansprüche 1 bis 10, **dadurch gekennzeichnet, dass** man überschüssiges Organylierungsmittel in einer Katalysatorzusammensetzung K1 durch Zugabe einer wirksamen Menge eines teilchenförmigen Feststoffs mit Hydroxylfunktionen oder adsorbiertem Wasser zersetzt, den Feststoff von der erhaltenen bei Bedarf mit einem Lösungsmittel verdünnten Katalysatorzusammensetzung K2 abtrennt und so eine Katalysatorzusammensetzung K4 erhält.

13. Verfahren nach einem der Ansprüche 1 bis 12, **dadurch gekennzeichnet, dass** man eine von Lösungsmittel und anorganischen Bestandteilen zumindest teilweise, bevorzugt im Wesentlichen vollständig befreite Katalysatorzusammensetzung K3 oder K4 verwendet, wobei die Überführung von K1 in K4 eine Zugabe von Wasser und eine Extraktion der wässrigen Lösung oder Suspension mit einem organischen Lösungsmittel umfasst.

14. Katalysatorzusammensetzung , insbesondere geeignet zur Durchführung von Oxidationsreaktionen nach einem der Ansprüche 1 bis 13, enthaltend mindestens ein Organylrheniumoxid der Formel RₐRe_{b}O_{c}L_{d}, worin R für einen Organylrest, a für eine ganze Zahl von 1 bis 6, b für eine ganze Zahl von 1 bis 4, c für eine ganze Zahl von 1 bis 12, d für eine ganze Zahl von 0 bis 4 und L für einen Lewis-basischen Liganden stehen und Re fünfbis siebenwertig, insbesondere aber siebenwertig ist, und vorliegend als Katalysatorzusammensetzung K1 oder einer daraus gewonnenen Katalysatorzusammensetzung K2 bis K4, **dadurch gekennzeichnet, dass** sie erhalten worden ist durch ein Verfahren, umfassend Organylierung einer Oxorheniumverbindung der allgemeinen Formel ReOₘAₙ, wobei Re fünf bis siebenwertig ist, A für eine anionische Abgangsgruppe, m für eine ganze Zahl 1, 2 oder 3 und n für eine ganze Zahl 0, 1, 2 oder 3 stehen, mit einer Grignardverbindung der Formel RMgCl, worin R für einen Organylrest, steht, und wobei pro Mol ReOₘAₙ 0,5 bis 10 Mol der Grignardverbindung eingesetzt werden, in einem aprotischen Lösungsmittelsystem in An- oder Abwesenheit einer Lewis-basischen Ligandenverbindung L unter Erhalt einer Katalysatorzusammensetzung K1, Zersetzung überschüssiger Grignardverbindung in der Katalysatorzusammensetzung K1 durch Zugabe eines protischen Mittels unter Erhalt einer Katalysatorzusammensetzung K2, mindestens teilweises Entfernen von Lösungsmitteln und/oder anorganischen Bestandteilen aus K1 beziehungsweise K2 unter Erhalt einer Katalysatorzusammensetzung K3 beziehungsweise K4, wobei die Herstellung von K1 bis K4 eine Sublimation, Kristallisation oder Destillation der darin enthaltenen Organylrheniumoxide der Formel RₐRe_{b}O_{c}L_{d} ausschließt und wobei bei der Herstellung der Katalysatorzusammensetzung ein Organylmagnesiumchlorid eingesetzt wurde, wobei Organyl ausgewählt ist aus der Reihe Methyl, Ethyl, n-Propyl, Isopropyl, n-Butyl, sek.-Butyl und Allyl.

15. Katalysatorzusammensetzung nach Anspruch 14, **dadurch gekennzeichnet, dass** bei ihrer Herstellung als Oxorheniumverbindung eine Verbindung aus der Reihe ReO₃, Re₂O₇, Hal-ReO₃, Hal₂ReO₂, ReO₃-O-Acyl, wobei Acyl eine ggf. fluorierte Alkanoylgruppe, Aryl-SO₂-, -P(O)(OR')₂, -Si(OR')₃, -P(O)R"₂ und -SiR"₃, worin R' und R" für Alkyl, Cycloalkyl, Arylalkyl oder Aryl stehen, die auch fluoriert sein können, eingesetzt wurde.

16. Katalysatorzusammensetzung nach einem der Ansprüche 14 oder 15, **dadurch gekennzeichnet, dass** pro Mol Oxorheniumverbindung 1 bis 5, insbesondere >1 bis 3 Mol Grignardverbindung eingesetzt wurden.

17. Katalysatorzusammensetzung nach einem der Ansprüche 14 bis 16, **dadurch gekennzeichnet, dass** sie zusätzlich mindestens eine zur Komplexbildung der in der Katalysatorzusammensetzung enthaltenen Organylrheniumoxide befähigte Stickstoffbase L enthält.

18. Katalysatorzusammensetzung nach einem der Ansprüche 14 bis 17, **dadurch gekennzeichnet, dass** es sich um eine von Lösungsmitteln und anorganischen Bestandteilen befreite Katalysatorzusammensetzung K3 oder K4 handelt.

## Claims

1. Process for oxidizing organic substrates, comprising reacting the organic substrate in a solvent system with a peroxo compound, in particular hydrogen peroxide, in the presence of an organylrhenium oxide of the formula RₐRe_{b}O_{c}L_{d}, where R is an organyl radical, a is an integer from 1 to 6, b is an integer from 1 to 4, c is an integer from 1 to 12, d is an integer from 0 to 4, L is a Lewis-basic ligand and Re is penta- to heptavalent, as a catalyst, **characterized in that** it utilizes a catalyst composition K1 or, obtained therefrom, a catalyst composition K2, K3 or K4, the preparation of K1 comprising the reaction of an oxorhenium compound of the general formula ReOₘAₙ, where Re is penta- to heptavalent, A is an anionic leaving group, m is 1, 2 or 3 and n is 0, 1, 2 or 3, with 1 to 10 organyl equivalents per mole of ReOₘAₙ of an organylating agent of an element selected from the group consisting of Li, Na, K, Mg, Al and Zn in an aprotic solvent in the presence or absence of a Lewis-basic ligand, the preparation of K2 comprising the addition of a protic agent to a composition K1 comprising excess organylating agent and the preparation of K3 and K4 comprising at least partial removal of solvents and/or inorganic constituents from K1 and K2 respectively, but the preparation of K1 to K4 excludes any sublimation, crystallization or distillation of the organylrhenium oxide or oxides of the formula RₐRe_{b}O_{c}L_{d} present therein.

2. Process according to Claim 1, **characterized in that** olefins as substrate are epoxidized or are hydroxylated to diols.

3. Process according to Claim 1, **characterized in that** amines as substrate are oxidized, in particular secondary amines are oxidized to disubstituted hydroxylamines.

4. Process according to Claim 1, **characterized in that** cyclic ketones are used as substrate and are oxidized to lactones by the Baeyer-Villiger method.

5. Process according to Claim 1, **characterized in that** aromatics, in particular polynuclear aromatics are used as substrate and are oxidized to quinones.

6. Process according to any one of Claims 1 to 5, **characterized in that** the oxorhenium compound used is a compound selected from the group consisting of ReO₃, Re₂O₇, Hal-ReO₃, Hal₂ReO₂, ReO₃-O-acyl, where Hal represents F, Cl, Br or I and acyl represents a group selected from the group consisting of R-C(O)-, aryl-SO₂-, -P(O)(OR')₂, -Si(OR')₃, -P(O)R"₂ and -SiR''₃, where R represents optionally fluorinated alkyl and R' and R" represent alkyl, cycloalkyl, arylalkyl or aryl, which can each also be fluorinated.

7. Process according to any one of Claims 1 to 6, **characterized in that** the organylating agent used is a compound selected from the group consisting of LiR, NaR, KR, MgR₂, RMgHal, ZnR₂, AlR₃ and Al(O)R, where R represents linear, branched or cyclic alkyl, in particular unbranched C₁-C₄ alkyl, benzyl or aryl, which can be alkyl substituted.

8. Process according to any one of Claims 1 to 6, **characterized in that** the organylating agent used is a compound selected from the group consisting of lithium alkyl compounds and Grignard reagents, in particular an alkylmagnesium halide and more preferably methylmagnesium chloride.

9. Process according to any one of Claims 1 to 8, **characterized in that** 1 to 5, in particular > 1 to 3 organyl equivalents are employed per mole of oxorhenium compound.

10. Process according to any one of Claims 1 to 9, **characterized in that** the organylation of the oxorhenium compound and/or the oxidation reaction are carried out in the presence, as a ligand L, of a nitrogen base which can form complexes of the formula RₐRe_{b}O_{c}L_{d} with the organylrhenium oxides present in the catalyst composition.

11. Process according to any one of Claims 1 to 10, **characterized in that** excess organylating agent present in the reaction mixture K1 is decomposed by adding an at least stoichiometric amount of a solvent selected from the group consisting of water, alcohols, carboxylic acids, silicas, boric acids and mineral acids, forming the catalyst composition K2.

12. Process according to any one of Claims 1 to 10, **characterized in that** excess organylating agent in a catalyst composition K1 is decomposed by adding an effective amount of a particulate solid with hydroxyl functions or adsorbed water, separating the solid from the resulting catalyst composition K2, which has been diluted with a solvent if necessary, thereby obtaining a catalyst composition K4.

13. Process according to any one of Claims 1 to 12, **characterized in that** it utilizes a catalyst composition K3 or K4 which is at least partly, preferably essentially completely, freed from solvent and inorganic constituents, the conversion of K1 into K2 comprising an addition of water and an extraction of the aqueous solution or suspension with an organic solvent.

14. Catalyst composition, in particular suitable for carrying out oxidation reactions according to any one of Claims 1 to 13, comprising at least one organylrhenium oxide of the formula RₐRe_{b}O_{c}L_{d}, where R is an organyl radical, a is an integer from 1 to 6, b is an integer from 1 to 4, c is an integer from 1 to 12, d is an integer from 0 to 4 and L is a Lewis-basic ligand and Re is penta- to heptavalent, but in particular heptavalent, and present as a catalyst composition K1 or, obtained therefrom, a catalyst composition K2 to K4, **characterized in that** it has been obtained by a process comprising organylating an oxorhenium compound of the general formula ReOₘAₙ, where Re is penta- to heptavalent, A is an anionic leaving group, m is an integer 1, 2 or 3 and n is an integer 0, 1, 2 or 3, with a Grignard compound of the formula RMgCl, where R is an organyl radical, using 0.5 to 10 mol of the Grignard compound per mole of ReOₘAₙ, in an aprotic solvent system in the presence or absence of a Lewis-basic ligand compound L, to obtain a catalyst composition K1, decomposition of excess Grignard compound in the catalyst composition K1 by addition of a protic agent to obtain a catalyst composition K2, at least partial removal of solvents and/or inorganic constituents from K1 and K2 respectively, to obtain a catalyst compositi0on K3 or K4 respectively, the preparation of K1 to K4 excluding any sublimation, crystallization or distillation of the organylrhenium oxides of the formula RₐRe_{b}O_{c}L_{d} present therein, and the preparation of the catalyst composition utilizes an organylmagnesium chloride wherein organyl is selected from the group consisting of methyl, ethyl, n-propyl, isopropyl, n-butyl, sec-butyl and allyl.

15. Catalyst composition according to Claim 14, **characterized in that** it was prepared using as oxorhenium compound a compound selected from the group consisting of ReO₃, Re₂O₇, Hal-ReO₃, Hal₂ReO₂, ReO₃-O-acyl, where acyl is an optionally fluorinated alkanoyl group, aryl-SO₂-, -P(O)(OR')₂, -Si(OR')₃, -P(O)R"₂ and -SiR"₃, where R' and R" represent alkyl, cycloalkyl, arylalkyl or aryl, which can each also be fluorinated.

16. Catalyst composition according to either of Claims 14 and 15, **characterized in that** 1 to 5, in particular > 1 to 3 mol of Grignard compound are used per mole of oxorhenium compound.

17. Catalyst composition according to any one of Claims 14 to 16, **characterized in that** it further comprises at least one nitrogen base L capable of complexing the organylrhenium oxides present in the catalyst composition.

18. Catalyst composition according to any one of Claims 14 to 17, **characterized in that** it comprises a catalyst composition K3 or K4 freed from solvents and inorganic constituents.

## Revendications

1. Procédé pour l'oxydation de substrats organiques, comprenant la transformation du substrat organique dans un système de solvants avec un composé de type peroxo, en particulier le peroxyde d'hydrogène, en présence d'un oxyde d'organylrhénium de formule RₐRe_{b}O_{c}L_{d}, où R représente un radical organyle, a représente un nombre entier de 1 à 6, b représente un nombre entier de 1 à 4, c représente un nombre entier de 1 à 12, d représente un nombre entier de 0 à 4, L représente un ligand basique de Lewis et Re est pentavalent à heptavalent, comme catalyseur, **caractérisé en ce qu'**on utilise une composition de catalyseur K1 ou une composition de catalyseur K2, K3 ou K4 obtenue à partir de celle-ci, la préparation de K1 comprenant la transformation d'un composé de type oxorhénium de formule générale ReOₘAₙ, où Re est pentavalent à heptavalent, A représente un groupe partant anionique, m représente le nombre 1, 2 ou 3 et n représente le nombre 0, 1, 2 ou 3, avec 1 à 10 équivalents organyle par mole de ReOₘAₙ d'un agent d'organylation d'un élément de la série Li, Na, K, Mg, Al et Zn dans un solvant aprotique en présence ou en l'absence d'un ligand basique de Lewis, la préparation de K2 comprenant l'addition d'un agent protique à une composition K1 contenant un agent d'organylation en excès et la préparation de K3 ou K4, respectivement, comprenant l'élimination au moins partielle des solvants et/ou des constituants inorganiques de K1 ou K2, respectivement, la préparation de K1 à K4 excluant toutefois une sublimation, une cristallisation ou une distillation du ou des oxydes d'organylrhénium de formule RₐRe_{b}O_{c}L_{d} qui est/sont contenus.

2. Procédé selon la revendication 1, **caractérisé en ce qu'**on époxyde ou qu'on hydroxyle en diols des oléfines en tant que substrat.

3. Procédé selon la revendication 1, **caractérisé en ce qu'**on oxyde des amines comme substrat, en particulier des amines secondaires, en hydroxylamines disubstituées.

4. Procédé selon la revendication 1, **caractérisé en ce qu'**on utilise, comme substrat, des cétones cycliques et qu'on les oxyde selon Baeyer-Villiger en lactones.

5. Procédé selon la revendication 1, **caractérisé en ce qu'**on utilise, comme substrat, des aromatiques, en particulier des aromatiques à plusieurs noyaux, et qu'on les oxyde en quinones.

6. Procédé selon l'une quelconque des revendications 1 à 5, **caractérisé en ce qu'**on utilise comme composé de type oxorhénium un composé de la série ReO₃, Re₂O₇, Hal-ReO₃, Hal₂ReO₂, ReO₃-O-acyle, où Hal représente F, Cl, Br ou I et acyle représente un groupe de la série R-C(O)-, aryl-SO₂-, -P(O)(OR')₂, -Si(OR')₃, -P(O)R''₂ et -SiR''₃, où R représente alkyle le cas échéant fluoré et R' et R'' représentent alkyle, cycloalkyle, arylalkyle ou aryle, qui peuvent également être fluorés.

7. Procédé selon l'une quelconque des revendications 1 à 6, **caractérisé en ce qu'**on utilise comme agent d'organylation un composé de la série LiR, NaR, KR, MgR₂, RMgHal, ZnR₂, AlR₃ et Al(O)R, où R représente un alkyle linéaire ramifié ou cyclique, en particulier un alkyle en C₁ à C₄ non ramifié, benzyle ou aryle, qui peut être substitué par alkyle.

8. Procédé selon l'une quelconque des revendications 1 à 6, **caractérisé en ce qu'**on utilise comme agent d'organylation un composé de la série des composés de type lithium-alkyle et des réactifs de Grignard, en particulier un halogénure d'alkylmagnésium et de manière particulièrement préférée le chlorure de méthylmagnésium.

9. Procédé selon l'une quelconque des revendications 1 à 8, **caractérisé en ce qu'**on utilise par mole de composé de type oxorhénium 1 à 5, en particulier > 1 à 3 équivalents organyle.

10. Procédé selon l'une quelconque des revendications 1 à 9, **caractérisé en ce qu'**on réalise l'organylation du composé de type oxorhénium et/ou la réaction d'oxydation en présence d'une base azotée comme ligand L qui peut former des complexes de formule RₐRe_{b}O_{c}L_{d} avec les oxydes d'organylrhénium contenus dans la composition de catalyseur.

11. Procédé selon l'une quelconque des revendications 1 à 10, **caractérisé en ce qu'**on décompose l'agent d'organylation en excès contenu dans le mélange réactionnel K1 par addition d'une quantité au moins stoechiométrique d'un solvant de la série eau, alcools, acides carboxyliques, acides siliciques, acides boriques et acides minéraux, la composition de catalyseur K2 étant formée.

12. Procédé selon l'une quelconque des revendications 1 à 10, **caractérisé en ce qu'**on décompose l'agent d'organylation en excès dans une composition de catalyseur K1 par addition d'une quantité active d'un solide en forme de particules avec des fonctions hydroxyle ou de l'eau adsorbée, on sépare le solide de la composition de catalyseur K2 obtenue, si nécessaire diluée par un solvant et on obtient ainsi une composition de catalyseur K4.

13. Procédé selon l'une quelconque des revendications 1 à 12, **caractérisé en ce qu'**on utilise une composition de catalyseur K3 ou K4 libérée au moins partiellement, de préférence de manière essentiellement complète, des solvants et des constituants organiques, la transformation de K1 en K4 comprenant une addition d'eau et une extraction de la solution ou suspension aqueuse avec un solvant organique.

14. Composition de catalyseur, convenant en particulier pour réaliser des réactions d'oxydation selon l'une quelconque des revendications 1 à 13, contenant au moins un oxyde d'organylrhénium de formule PₐRe_{b}O_{c}L_{d}, R représentant un radical organyle, a représentant un nombre entier de 1 à 6, b un nombre entier de 1 à 4, c un nombre entier de 1 à 12, d un nombre entier de 0 à 4 et L un ligand basique de Lewis et Re étant pentavalent à heptavalent, en particulier toutefois heptavalent, et se trouvant sous forme de composition de catalyseur K1 ou d'une composition de catalyseur K2 à K4 pouvant être obtenue à partir de celle-ci, **caractérisée en ce qu'**elle a été obtenue par un procédé comprenant l'organylation d'un composé de type oxorhénium de formule générale ReOₘAₙ, où Re est pentavalent à heptavalent, A représente un groupe partant anionique, m représente un nombre entier 1, 2 ou 3 et n représente un nombre entier 0, 1, 2 ou 3 avec un composé de Grignard de formule RMgCl, où R représente un radical organyle et où on utilise par mole de ReOₘAₙ 0,5 à 10 moles du composé de Grignard, dans un système de solvants aprotiques, en présence ou en l'absence d'un composé de type ligand basique de Lewis L avec obtention d'une composition de catalyseur K1, la décomposition du composé de Grignard en excès dans la composition de catalyseur K1 par addition d'un agent protique avec obtention d'une composition de catalyseur K2, l'élimination au moins partielle des solvants et/ou des constituants inorganiques de K1 ou K2, respectivement, avec l'obtention d'une composition de catalyseur K3 ou K4, respectivement, la préparation de K1 à K4 excluant une sublimation, une cristallisation ou une distillation des oxydes d'organylrhénium de formule RₐRe_{b}O_{c}L_{d} qui y sont contenus et où on utilise, lors de la préparation de la composition de catalyseur, un chlorure d'organylmagnésium, l'organyle étant choisi dans la série méthyle, éthyle, n-propyle, isopropyle, n-butyle, sec-butyle et allyle.

15. Composition de catalyseur selon la revendication 14, **caractérisée en ce qu'**on utilise, lors de sa préparation, comme composé de type oxorhénium, un composé de la série ReO₃, Re₂O₇, Hal-ReO₃, Hal₂ReO₂, ReO₃-O-acyle, où acyle représente un groupe alcanoyle le cas échéant fluoré, aryl-SO₂-, -P(O)(OR')₂, -Si(OR')₃, -P(O)R"₂ et -SiR''₃, où R' et R" représentent alkyle, cycloalkyle, arylalkyle ou aryle, qui peuvent également être fluorés.

16. Composition de catalyseur selon l'une quelconque des revendications 14 ou 15, **caractérisée en ce qu'**on utilise par mole de composé de type oxorhénium 1 à 5, en particulier > 1 à 3 moles de composé de Grignard.

17. Composition de catalyseur selon l'une quelconque des revendications 14 à 16, **caractérisée en ce qu'**elle contient en outre au moins une base azotée L apte à la formation d'un complexe avec les oxydes d'organylrhénium contenus dans la composition de catalyseur.

18. Composition de catalyseur selon l'une quelconque des revendications 14 à 17, **caractérisée en ce qu'**il s'agit d'une composition de catalyseur K3 ou K4 libérée des solvants et des constituants inorganiques.
